# EUROPEAN PATENT APPLICATION

(11) **EP 4 015 010 A1**
(43) Date of publication of application: **22.06.2022**
(21) Application number: 20214568.6
(22) Date of filing: 16.12.2020
(51) Int. Cl.: A61M 1/06

(54) **BREAST PUMP SYSTEM**

(71) Applicant: Koninklijke Philips N.V., 5656 AG Eindhoven (NL)
(72) Inventor: VAN VELDHUIZEN, Gijsbert Hendrik, 5656 AE Eindhoven (NL); CLAASSEN, Coen Petrus Martinus, 5656 AE Eindhoven (NL); DOBRUSSKIN, Christoph, 5656 AE Eindhoven (NL); BROCKHUIS, Lili-Marjan, 5656 AE Eindhoven (NL); VAN DEN BIJGAART, Adrianus Wilhelmus Dionisius Maria, 5656 AE Eindhoven (NL)
(74) Representative: Philips Intellectual Property & Standards

(57) **Abstract**

A breast pump system has first and second expression kits, and they have different sets of functional units. However, they have shape features which are designed to provide similar appearance in use, to make the use of the breast pump system more discrete.

## Description

### FIELD OF THE INVENTION

This invention relates to breast pump systems, in particular with an expression kit for each breast.

### BACKGROUND OF THE INVENTION

Breast pumps are used by breast feeding women to extract milk such that the extracted milk can be fed to their babies at a later time.

Typically, the breast is placed into a funnel shaped cup and a vacuum is applied such that milk is extracted. The breast pump typically has two operating modes, namely a stimulation mode and an extraction mode. During the stimulation mode, the milk ejection reflex is stimulated. When the breast pump is activated, a pressure source such as a vacuum pump inside the breast pump generates a vacuum and the stimulation mode can be started. The vacuum pump is typically driven by an electric motor. For portable breast pumps, these motors are driven by battery power.

Wearable breast pumps are characterized in that they can be fully worn on the user's body, allowing the user to move around freely. Some of these are small enough to fit into a user's bra. A motor, battery and milk container are for example integrated to form a single unit.

Often, it is desirable to express both breasts at the same time. A user will then typically acquire a set of identical devices to achieve a setup which performs stimulation and expression independently for each breast. By having two similar-shaped devices a limited impact on level of discreteness is achieved as the additional volume for each breast is similar. However, this gives a high purchase price and there is no sharing of functions between the two devices.

### SUMMARY OF THE INVENTION

The invention is defined by the claims.

According to examples in accordance with an aspect of the invention, there is provided a breast pump system comprising:
a first expression kit; and
a second expression kit,
wherein the first and second expression kits each have a different respective set of functional units which thereby define a different set of functional capabilities of the expression kit, and the first and second expression kits have matching outer shapes.

This breast pump system makes use of two expression kits, but they have different functional capabilities. Thus, the set of functional parts is different between the two kits. In this way, the cost of the overall system can be kept to a minimum by avoiding unnecessary duplication of functions.

The differences between the expression kits may reside in the hardware components and/or the software functionality of a controller.

The first and second expression kits are designed to avoid a visually perceivable difference in size or shape, i.e. a visually matching outer shape, by having substantially matching outer designs. This means that when in use, the two expression kits look like a matching pair. They may have identical or mirror symmetrical outer shape. They may have approximately the same internal volume so that the volume added by wearing each expression kit is the same. For example, the added volume of one expression kit may be in the range 1.0 to 1.1 times the added volume of the other.

The first and second expression kits for example have a same cup size for fitting to a feeding bra. The first and second expression kits may also have the same cup size for fitting to the mother, although it is equally possible for the two expression kits to have a different (internal) cup size for fitting to the mother but a same external cup size (for mothers with different sized breasts). The two expression kits for example are intended to sit with the same or similar angle relative to the vertical and project forwardly from the breast by a same or similar amount.

The first and second expression kits may each have a respective set of functional units, including a different combination of one of more features selected from the list:
a battery;
an electrical pump;
a mechanical system for generating a constant baseline under-pressure;
a system for generating a pressure waveform during a stimulation mode for triggering the milk ejection reflex;
a system for generating a pressure waveform during an expression mode;
a system for generating a pressure waveform during a massage mode;
a milk collection vessel;
a user input interface for receiving user commands;
a user output interface for providing status information to a user;
a communications interface for communicating wirelessly with an external device;
a milk sensor;
a timer; and
an ambient condition sensor,

By way of example, a stimulation and expression device may be combined with an expression-only device so that better value can be achieved, as the price of the overall setup is lower. This may for example be possible by using the MER stimulation for one breast to trigger the MER in both breasts, because it is a hormone based trigger not specific to one breast. However, the solution remains discrete because the outer appearance (in particular the outer shape) of the two devices match. They may for example be worn in a feeding bra with the breast feeding being less obvious to a third party.

Each expression kit is preferably attached to a respective milk collecting vessel. The expression kit and its respective milk collecting vessel preferably fit in the cup of a breast feeding bra, thus providing a discrete breast-feeding solution.

The system for generating a pressure waveform during a stimulation mode for example comprises a valve and a controller for controlling the valve. This valve is then used in conjunction with an electric vacuum pump to provide a regulated pressure waveform.

The system for generating a pressure waveform during an expression mode may also comprise a valve and a controller for controlling the valve. The control implemented by the controller will be different for the stimulation mode and for the expression mode, for example with different cyclic frequency and optionally also different maximum under-pressure level (i.e. the amount by which the pressure is below atmospheric pressure). Thus, the differences may reside in the control software.

In one example, only one of the first and second expression kits has a controller for generating the pressure waveform during a stimulation mode. Thus, only one kit is used to stimulate the MER. This creates a hormone response which influences both breasts. Thus, the other kit does not need to have the functionality of the stimulation mode.

In another example, only one of the first and second expression kits has a user input interface and a user output interface. Thus, any interaction with the user (e.g. advising of battery life, giving information about milk flow etc.) can be implemented in one expression kit only.

In another example, only one of the first and second expression kits has an ambient condition sensor. Sensing at only one location is sufficient for many ambient sensing functions. For example, the ambient condition sensor is a noise sensor.

In another example, only one of the first and second expression kits has a communications interface for communicating wirelessly with an external device. This external device may be a mobile phone which provides an interface to the user.

The first and second expression kits may for example be coupled together by a wired connection so that the feedback to the user can nevertheless provide status information about both expression kits.

In another example, only one of the first and second expression kits has an electrical pump, and the other has a mechanical system for generating a constant baseline under-pressure. This mechanical system for example comprises an element to be squeezed or pressed by the user to generate an under-pressure, which is then retained.

The first and second expression kits may each be configurable by a user to select a desired set of functional units. This provides a modular system.

One or both expression kits may have a battery. When only one has a battery, the other may be supplied with power by a wired connection between them.

Another aspect of the invention provides an expression kit for a breast pump system, comprising:
a housing; and
a set of functional units for mounting to the housing,
wherein at least some of the functional units are modular such that the set of functional units is configurable to implement any desired functionality from the list:
   stimulation and expression;
   stimulation and not expression;
   expression and not stimulation.

The mounting "to" the housing may be in the housing or on the outside of the housing.

The set of functional units is for example configurable to additionally provide massaging functionality and/or local milk collection functionality.

The set of functional units is for example selected from the list:
a battery;
an electrical pump;
a mechanical system for generating a constant baseline under-pressure;
a system for generating a pressure waveform during a stimulation mode for triggering the milk ejection reflex;
a system for generating a pressure waveform during an expression mode;
a system for generating a pressure waveform during a massage mode;
a milk collection vessel;
a user input interface for receiving user commands;
a user output interface for providing status information to a user;
a communications interface for communicating wirelessly with an external device;
a milk sensor;
a timer; and
an ambient condition sensor,

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows a known breast pump system;
Figure 2 shows a mother wearing two wearable breast pumps;
Figure 3 shows a breast pump system in accordance with the invention;
Figure 4 shows one example implementation of the system of Figure 3; and
Figure 5 shows examples of dimensions which enable the use of two similar expression kits to be discrete.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a breast pump system which has first and second expression kits, and they have different sets of functional units. However, they have shape features which are designed to provide similar appearance in use, to make the use of the breast pump system more discrete.

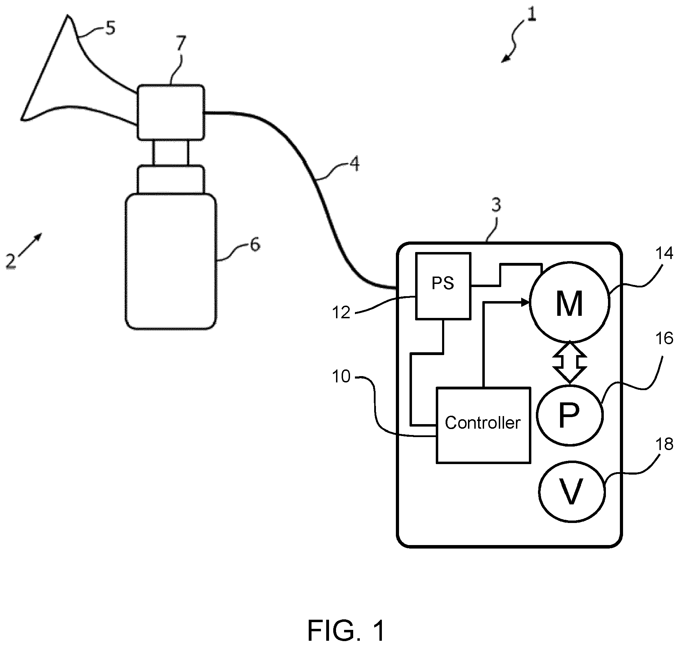

Figure 1 shows a known breast pump system 1, comprising an expression unit 2 and a pump arrangement 3 which are connected via a tube 4. The pump arrangement includes various components in addition to the pump, so may be considered to be a general operating unit.

The expression unit 2 is formed with a main body 7, a funnel 5 for receiving a breast of a user and a receptacle 6 for collecting the expressed milk. The funnel 5 and the receptacle 6 are connected to the main body 7. The main body 7 comprises a vacuum chamber. A flexible membrane or diaphragm is located in the vacuum chamber. The membrane prevents expressed milk from flowing into the tube 4 leading to the pump arrangement unit 3.

The pump arrangement 3 may instead be directly mounted and connected to the main body 7. In this case, the membrane prevents expressed milk from flowing directly into the pump arrangement 3.

The pump arrangement 3 comprises a controller 10, a power source 12, a motor 14 and a vacuum pump 16. The controller controls 10 the operation of the power source 12, motor 14 and vacuum pump 16. The pump arrangement 3 further comprises a vacuum release component, such as a solenoid valve 18.

In use, the vacuum pump applies a vacuum to the membrane located in the main body 7 so that it deforms. The membrane deforms to create a vacuum in the funnel 5 which in turns applies a vacuum to the breast which enables milk to be expressed. Although the breast pump system 1 is described as comprising a membrane such that the vacuum is applied indirectly to the breast, it should be understood that in an alternative embodiment, the vacuum is applied directly to the breast of a user. In this case, the breast pump system does not comprise a membrane and the vacuum created by the vacuum pump is applied directly to the breast.

The vacuum is applied to the breast at intervals. That is, a pressure differential is applied on a cyclic basis. After a vacuum has been established, the pressure from the vacuum is released by the use of the solenoid valve which is temporarily opened. The solenoid valve is an electromechanically operated valve configured to open and close an air passage that connects to the vacuum side of the vacuum pump to ambient air such that when the solenoid valve is closed, the vacuum pump generates a vacuum in the expression unit which enables milk to be expressed from the breast of a user. When the solenoid valve is opened, the vacuum generated by the vacuum pump is released as ambient air flows towards the vacuum or negative pressure created by the vacuum pump such that the pressure exerted on the breast of a user is partially or completely reduced.

This is a basic description of the known operation of a standard breast pump system.

There are also wearable breast pumps, which are for example for mounting within a feeding bra. All of the drivetrain components described above are then formed within an outer enclosure which fits over the breast. In one type of design, a top part contains the drivetrain (pump, motor, controller) and a bottom part forms the collection vessel. This enables breast pumping to be performed more discretely. Of course, other designs are possible.

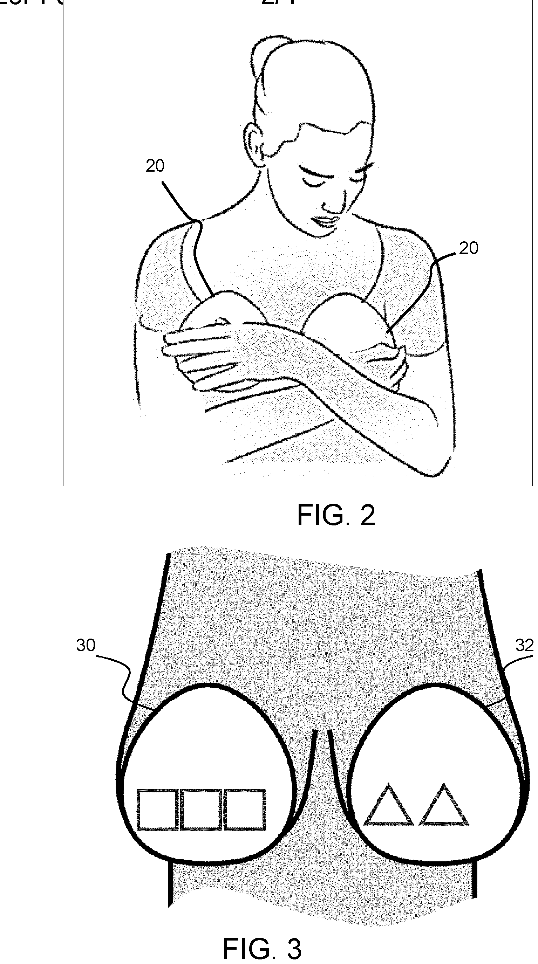

Figure 2 shows a mother wearing two wearable breast pumps 20.

In such a design, the funnel 5 of Figure 1 is implemented as breast shield, and this breast shield is received within an outer enclosure of the wearable breast pump.

Figure 3 shows a breast pump system comprising a first expression kit 30 and a second expression kit 32. Each expression kit is a wearable device, and each expression kit is fitted over a breast shield.

The expression kits may each comprise a milk sensor for sensing milk flow and for measuring flow volume.

The first expression kit 30 has a respective first set of functional units, represented by rectangles. The second expression kit 32 has a respective second set of functional units, represented by triangles. The sets are different although there may be some elements repeated between the sets. For example, each set of functional units includes a breast shield (not shown).

There are various other functional units which can be used to make up the sets.

An expression kit may use electrical pumping, using an electrical pump and motor as explained above, and the pump pressure profile may be controlled by a controller in combination with a pressure regulating valve. Alternatively, a mechanical system may be used for generating a constant baseline under-pressure.

If the milk ejection reflex is triggered in one breast using a particular cyclic pressure waveform during a stimulation mode, the expression of the other breast may simply use a baseline vacuum pressure.

Different controller functions may be implemented, such as the stimulation mode mentioned above and an expression mode using a different cyclic waveform. The controller may also be implemented with a massage mode.

Other functional units include a user input interface for receiving user commands, a user output interface for providing status information to a user, a timer, and a communications interface for communicating wirelessly with an external device such as a mobile telephone. Sensors may also be used, such as a noise sensor or other ambient condition sensors.

A noise sensor is for example used to improve discreetness of the device. For example, the noise level can be reduced (with a consequent decrease in device performance) when a low environmental noise level is detected, in order to maintain discreetness in a smart manner.

The expression kit may include a local milk collection vessel, or it may connect to a remote milk collection vessel. Thus, the expression kit (i.e. the part located over the breast) may include a milk collection vessel or not.

Milk sensing may also be used (in one or both expression kits) to provide feedback to the mother about the milk flow.

As explained above, the first set of functional units is different to the second set of functional units. Thus, the two expression kits have different overall functionality. For example, one may be a master device with a first range of functions, and the other may be a slave device with a reduced second range of functions, wherein functions of the first device which do not need to be duplicated are not present in the second device.

Examples of functions which do not need to be duplicated are:
a battery, because there can be a power connection between the two kits, or the second kit may even be entirely manual;
a controller with a stimulation mode function, because the MER only needs to be triggered in one device;
a user input and/or output interface;
a communication system to a remote device such as a mobile phone;
a pressure regulating valve and associated controller, because the second kit may simply use a static baseline pressure.

The first and second functional units however have matching outer designs.

What is meant by this is that the two expression kits look the same shape when in use. They may have different visual (surface decoration) appearance so the user knows which is which (since they are covered by clothing in normal use), but the form factors (i.e. the shapes but allowing for a possible left-right mirror imaging) are preferably the same, or sufficiently similar that a third party seeing the two kits in use would not perceive a difference in size or shape.

This breast pump system thus makes use of two expression kits, but they have different functional capabilities. In this way, the cost of the overall system can be kept to a minimum by avoiding unnecessary duplication of functions.

The expression kits may have identical shapes, or they may have mirror symmetrical outer shapes (relative to each other). Some the shape parameters which may be designed to equalize and protect discreetness are discussed further below.

One configuration of particular interest is to provide a stimulation and expression kit and a lower cost expression-only kit, so that better value can be achieved. The expression only kit may still have a controller and a pressure regulating valve, but this may be a reduced functionality controller. Instead, the expression only kit may not need to implement a cyclic pressure waveform at all - it may simply implement a constant baseline vacuum. This may be achieved with a pump with no need for a pressure regulating valve, or it may even be achieved with a mechanical system.

When a cyclic waveform is used, different vacuum levels are applied over time. The controller may control a pressure regulating valve, as mentioned above, but it could instead control the drive signal to the pump motor.

When there are cyclic expression and stimulation modes, they for example have different cyclic frequency and different maximum under-pressure (i.e. vacuum) levels.

The expression mode for example cycles between a baseline vacuum and a maximum vacuum (i.e. a maximum under-pressure) "Max_Vac".

There is first a time to (maximum) vacuum, TTV, then a dwell-in time DI, then a time to atmosphere (or to the baseline vacuum), TTA, and finally a dwell-out time, DO. The vacuum rate is defined as Max_Vac/TTV and the atmospheric rate is defined as Max_Vac/TTA.

By way of example:
Baseline Vac = atmospheric pressure
Max Vac = 300 mbar (30 kPa) below atmospheric pressure
TTV = 0.85 s
DI = 0.3 s
TTA = 0.05 s
DO = 0.35 s

This gives a total cycle time T_cycle of 1.55s.

These values are simply by way of example. The maximum under-pressure may be higher, for example 350 mbar (35 kPa) and the durations may differ. The cycle time is generally of the order of 1 to 2 seconds.

The stimulation mode for example has a faster frequency hence with a shorter cycle time of around 0.5 seconds, and a lower vacuum, for example a maximum under-pressure level of 150 mbar (15 kPa).

In an expression kit with both stimulation mode and expression modes, the stimulation mode for example takes placing during an initial period of the breast pumping session (e.g. 50 seconds) and the expression mode takes place during the remainder of the breast pumping session.

A controller may also implement a positioning mode. This may comprise intermittent application of a small under-pressure to allow the user to position the breast shield while observing the correct alignment. A controller may also implement a massage mode.

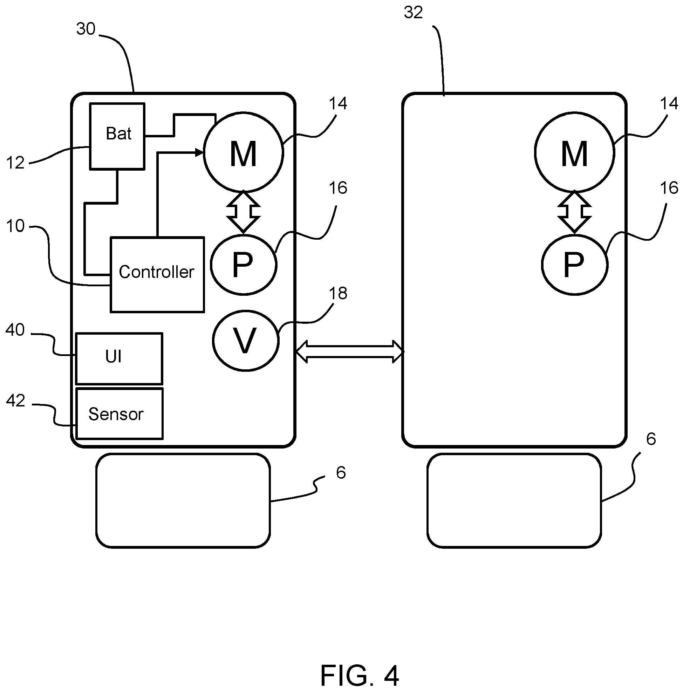

Figure 4 shows an example of an implementation of the system of Figure 3.

A first expression kit 30 has a controller 10, battery 12, motor 14, pump 16 and pressure regulating valve 18. The controller 10 implements a stimulation mode and an expression mode. The first kit 30 also has a user interface 40 (input and output) and a sensor 42.

A second expression kit 30 has motor 14 and pump 16, but power and control commands are received over a wired link to the first kit 30. There is only an expression mode of a constant baseline pressure so no regulating valve is present. The pump motor may, however, be controlled by the first kit to select the baseline pressure level. The second kit is a slimmed down version with no user interface, sensing or power supply. The wired connection does mean however that feedback to the user can nevertheless provide status information about both expression kits.

The second kit may even have a purely mechanical system for generating a constant baseline under-pressure. This mechanical system for example comprises an element to be squeezed or pressed by the user to generate an under-pressure, which is then retained.

The first and second expression kits may each be configurable by a user to select a desired set of functional units. This provides a modular system.

As explained above, the outer shapes are the same or mirror images of each other, for example by having outer shells with a design having similar form factor. The shells may then be used with different functional units as discussed above. A user then buys two or more shells and equips them with different functionality for example to achieve lower cost or for suitability in different use scenarios (e.g. home, work, car etc.).

The first kit in particular may be used on its own. By enabling the functional units to be swapped between shells (which are typically mirror symmetric rather than identical), the full functionality may be fitted to the left shell or the right shell.

In a modular design, the expression kit has a housing and a set of functional units for mounting to (typically in) the housing. The various possible functional units are explained above. At least some of the functional units are modular such that the set of functional units may be selected to implement desired combinations of functions.

As explained above, expression and stimulation are different functions, although the difference may reside in software rather than hardware.

Thus, the modules may be configurable to enable stimulation and expression or stimulation and not expression (in which case the other breast may be expressing) or expression and not stimulation (in which case stimulation may be applied to the other breast).

In further examples, any of the (sixteen) possible combinations of the following functions may configurable:
stimulation functionality;
expression functionality;
massaging functionality; and
local milk collection functionality.

As explained above, the expression kits are designed to be discrete in use, in particular so that a third party will not visually perceive the presence of the breast pump because there is no immediately obvious asymmetry between the two sides.

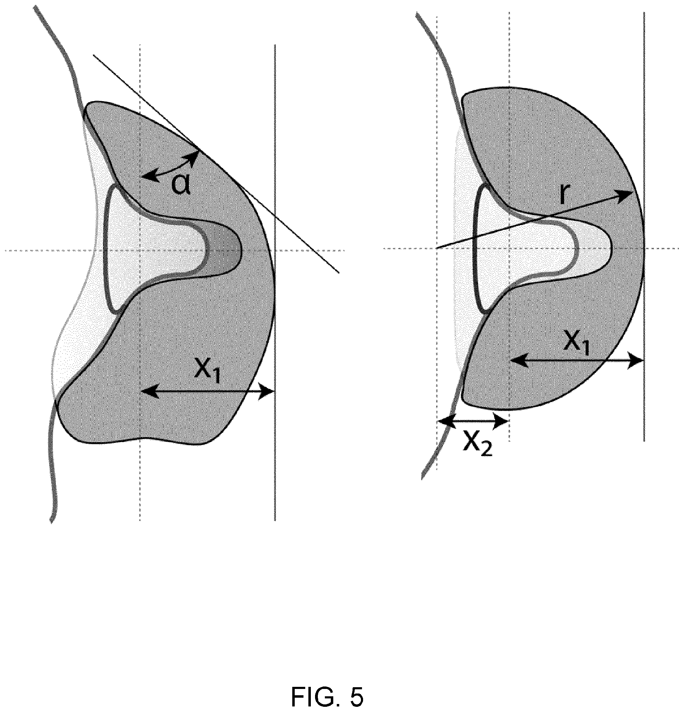

Figure 5 shows some of the design parameters which are important for achieving a matching visual appearance. The left image shows a cross section through a vertical plane (a side view) and the right image shows a cross section through a horizontal plane (a top view).

The dimension X₁ is the amount by which the front of the expression kit extends beyond the front of the breast, i.e. the added thickness.

From above, the front surface is generally circular with radius r. The dimension X₂ is the offset of the center of that circle behind the back of the nipple.

The angle α represent a top surface angle to the vertical.

Each of these parameters may be matched, for example in each case, they may the same, or the larger of the two is in the range of 1.0 to 1.1 times the smaller of the two. The outer surface of each expression kit may correspond to a cup size for fitting to a feeding bra, and the cup sizes are the same. The added volume (i.e. between the breast and the feeding bra) may also be the same, or in the 1.0 to 1.1 range.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to".

Any reference signs in the claims should not be construed as limiting the scope.

## Claims

1. A breast pump system comprising:
a first expression kit (30); and
a second expression kit (32),
wherein the first and second expression kits each have a different respective set of functional units which thereby define a different set of functional capabilities of the expression kit, and the first and second expression kits have matching outer shapes.

2. The breast pump system of claim 1, wherein the first and second expression kits each have a respective set of functional units, including a different combination of one of more features selected from the list:
a battery (12);
an electrical pump (14, 16);
a mechanical system for generating a constant baseline under-pressure;
a system (12,18) for generating a pressure waveform during a stimulation mode for triggering the milk ejection reflex;
a system (12,18) for generating a pressure waveform during an expression mode;
a system (12,18) for generating a pressure waveform during a massage mode;
a milk collection vessel;
a user input interface (40) for receiving user commands;
a user output interface (40) for providing status information to a user;
a communications interface for communicating wirelessly with an external device;
a milk sensor;
a timer; and
an ambient condition sensor (42),

3. The system of claim 1 or 2, wherein the first and second expression kits are for fitting to a feeding bra with the same cup size.

4. The system of any one of claims 1 to 2, wherein the first and second expression kits have identical or mirror symmetrical outer shape.

5. The system of any one of claims 1 to 4, wherein each expression kit is attached to a respective milk collecting vessel.

6. The system of any one of claims 1 to 6, wherein the system for generating a pressure waveform during a stimulation mode or during an expression mode comprises a valve and a controller for controlling the valve.

7. The system of any one of claims 1 to 6, wherein only one of the first and second expression kits has:
a controller for generating a stimulation pressure waveform during a stimulation mode;
a user input interface and a user output interface; or
a noise sensor.

8. The system of any one of claims 1 to 7, wherein only one of the first and second expression kits has a communications interface for communicating wirelessly with an external device.

9. The system of claim 8, wherein the first and second expression kits are coupled together by a wired connection.

10. The system of any one of claims 1 to 9, wherein only one of the first and second expression kits has an electrical pump, and the other has a mechanical system for generating a constant baseline under-pressure.

11. The system of any one of claims 1 to 10, wherein the first and second expression kits are each configurable by a user to select a desired set of functional units.

12. The system of any one of claims 1 to 11, wherein the first and second expression kits are wearable.

13. An expression kit for a breast pump system, comprising:
a housing; and
a set of functional units for mounting to the housing,
wherein at least some of the functional units are modular such that the set of functional units is configurable to implement any desired functionality from the list:
stimulation and expression;
stimulation and not expression;
expression and not stimulation.

14. The expression kit of claim 13, wherein the set of functional units is configurable to additionally provide massaging functionality and/or local milk collection functionality.

15. The expression kit of claim 13 or 14, wherein the set of functional units is selected from the list:
a battery (12);
an electrical pump (14, 16);
a mechanical system for generating a constant baseline under-pressure;
a system (12,18) for generating a pressure waveform during a stimulation mode for triggering the milk ejection reflex;
a system (12,18) for generating a pressure waveform during an expression mode;
a system (12,18) for generating a pressure waveform during a massage mode;
a milk collection vessel;
a user input interface (40) for receiving user commands;
a user output interface (40) for providing status information to a user;
a communications interface for communicating wirelessly with an external device;
a milk sensor;
a timer; and
an ambient condition sensor (42).
